Europäisches Patentamt

European Patent Office

Office européen des brevets

(19)

(11) **EP 0 995 463 B1**

(12) **EUROPÄISCHE PATENTSCHRIFT**

(45) Veröffentlichungstag und Bekanntmachung des Hinweises auf die Patenterteilung:
**16.08.2001 Patentblatt 2001/33**

(51) Int Cl.[7]: **A61N 2/02**

(21) Anmeldenummer: **98119944.1**

(22) Anmeldetag: **21.10.1998**

(54) **Vorrichtung und elektrisches oder elektromagnetisches Signal zur Beeinflussung biologischer Abläufe**

Device applying electric or electromagnetic signals for promoting biological processes

Appareil appliquant des signaux électriques ou électromagnétiques pour influencer des processus biologiques

(84) Benannte Vertragsstaaten:
**AT BE CH DE DK ES FI FR GB GR IE IT LI LU MC NL PT SE**
Benannte Erstreckungsstaaten:
**SI**

(43) Veröffentlichungstag der Anmeldung:
**26.04.2000 Patentblatt 2000/17**

(73) Patentinhaber: **Kafka, Wolf. A., Prof.
82288 Kottgeisering (DE)**

(72) Erfinder: **Kafka, Wolf. A., Prof.
82288 Kottgeisering (DE)**

(74) Vertreter: **Grünecker, Kinkeldey,
Stockmair & Schwanhäusser Anwaltssozietät
Maximilianstrasse 58
80538 München (DE)**

(56) Entgegenhaltungen:
**EP-A- 0 266 907        WO-A-96/32158
DE-A- 4 221 739        US-A- 4 428 366
US-A- 5 181 902**

**Beschreibung**

Die Erfindung betrifft eine Vorrichtung zur Beeinflussung biologischer Abläufe in einem lebenden Gewebe, insbesondere einem menschlichen Körper, durch Beaufschlagung zumindest eines Teils des Gewebes mit einem pulsierenden elektromagnetischen Feld.

Seit Beginn der 70iger Jahre sind Vorrichtungen bekannt, die elektromagnetische Felder erzeugen und die routinemäßig in Kliniken, speziell im Bereich der Orthopädie, zu therapeutischen Zwecken eingesetzt werden. Zunächst wurden dabei semiinvasive Verfahren mit pulsierenden Magnetfeldern verwendet. Die sinusförmigen Magnetfelder wiesen eine Frequenz von 2 bis 20 Hz und magnetische Flussdichten zwischen 1mT und 10 mT auf. Nach dem Prinzip der Induktion wurden durch die externen Magnetfelder, die den behandelten Körperteil in seiner Längsachse durchfluten, aufgrund der zeitlichen Änderung des magnetischen Flusses an implantierten Elektroden mit Hilfe eines sog. Sekundärelementes eine Wechselspannung erzeugt. Die implantierten Elektroden wiesen zur Behandlung von Knochenbrüchen die Form eines Marknagels oder von Schrauben auf. Aber auch damals war schon die Möglichkeit einer nichtinvasiven Behandlung ohne implantiertem Sekundärelement bekannt, wobei allerdings im behandelten Körperteil, der sich im Zentrum der Spule zu befinden hatte, nur sehr schwache elektrische Ströme induziert wurden. Ebenfalls seit Beginn der 70iger Jahre sind Geräte für die Ganzkörpertherapie bekannt, wobei sich die Feldlinien gleichmäßig im Körper verteilen. Ein Gerät zur Elektrotherapie mit Impulsgenerator und Felderzeugungs vorrichtung wird im Dokument EP-A-0 266 907 beschrieben.

Alle bisher bekannten Vorrichtungen zur Behandlung des menschlichen Körpers führen jedoch nur selten zu der erwünschten beschleunigenden Wirkung des Heilungsvorgangs. Es ist insbesondere problematisch, daß bei bisherigen Vorrichtungen zur Erzielung eines deutlich beschleunigten Heilerfolges die Anwendung häufig wiederholt werden muss, was zu einer erhöhten Belastung der Patienten und im Ergebnis zu deutlich höheren Behandlungskosten führte.

Aufgabe der Erfindung ist es, eine Vorrichtung anzugeben, mit der eine schnellere und in ihrer physiologischen Wirkung breitere Beeinflussung, insbesondere Anregung, biologischer Abläufe ermöglicht wird.

Diese Aufgabe wird erfindungsgemäß dadurch gelöst, daß eine Vorrichtung geschaffen wird, die einen Impulsgenerator und eine Felderzeugungsvorrichtung zur Erzeugung eines pulsierenden elektromagnetischen Feldes umfasst. Der Impulsgenerator dient zur Ansteuerung der Felderzeugungsvorrichtung, wobei der Impulsgenerator die Felderzeugungsvorrichtung über geeignete Strom-Spannungsabläufe so ansteuert, daß das pulsierende elektrische oder elektromagnetische Feld aus einer Vielzahl von, hinsichtlich ihres zeitlichen Amplitudenverlaufs charakteristisch geformten Einzelimpulsen besteht, deren Frequenz zwischen 1 und 1000 Hz liegt. Ein derartiger Einzelimpuls kann sich dabei aus einer Überlagerung eines nach einer Potenzfunktion an- oder absteigenden Grundimpulses mit einer Reihe von aufgesetzten Pulsen von jeweils kürzerer Zeitdauer und unterschiedlicher Form und zeitlicher Abfolge aufbauen.

Der zeitliche Amplitudenverlauf eines derartigen Einzelimpulses könnte etwa der folgenden Funktion entsprechen:

$$y = \frac{x^a \cdot k \cdot e^{sin(x^b)}}{c} + d$$

Die Parameter dieser Formel beschreiben jeweils:

y die Amplitude des im Impulsgenerator erzeugten Spannungsverlaufs, x den Zeitverlauf, wobei die Zeit x für jeden einzelnen Impuls wieder von neuem mit demselben Anfangswert beginnt, a den zeitlichen Amplitudenverlauf jedes einzelnen Grundimpulses (Hüllkurve), b - in einer Art Dichtefunktion - die Anzahl und Flankensteilheit der überlagerten Pulse, k die Amplitude der überlagerten Pulse, c einen Faktor zur Einstellung der Amplitude und d einen Offsetwert.

Die gegenüber herkömmlichen Vorrichtungen verbesserte Vorrichtung führt zu einer deutlich schnelleren Anregung von Stoffwechselvorgängen in bestrahltem Gewebe. Das könnte darauf beruhen, daß die den Grundimpulsen überlagerten Pulse die physiologischen Austauschprozesse über intrakorporale Membransysteme verbessern, da die aufgesetzten Pulse nach dem Induktionsgesetz (der Maxwellschen Gleichungen) entsprechend ihrer speziellen Form, z.B. wachsenden Flankensteilheiten, höhere elektromagnetische Feldspitzen induzieren, die z.B. über die von ihnen ausgehenden elektromotorischen Kraftwirkungen die im allgemeinen hochselektiven physiochemischen Reaktionsmechanismen durch eine entsprechende breitbandige Absenkung der Aktivierungsenergien beeinflussen und so - vor allem in Membranbereichen - die physiologischen Austauschprozesse stimulierend wirken. Diese Stimulation führt insbesondere zu einer erhöhten $O_2$-Utilisation.

Besonders vorteilhaft an der erfindungsgemäßen Vorrichtung ist, daß sie auch bei einer lokalen Bestrahlung zu einer Anregung der Stoffwechselvorgänge im gesamten Körper also auch nicht bestrahlten Bereichen des Individuums führt.

Mit einer solchen Bestrahlung lassen sich vorteilhafte Wirkungen bei verschiedenen medizinischen Anwendungen erreichen. Eine erhöhte $O_2$-Utilisation führt unter anderem auf der einen Seite zu einer verstärkten Bindegewebes- und Knorpelbildung und einer zusätzlichen Vaskularisation.

Auf der anderen Seite kann, eventuell begünstigt durch die obige $O_2$-Utilisation, aufgrund der bioelektrischen Wirkung der induzierten Spannungen auch eine Mineralisation des Bindegewebes durch erhöhten lonenaustausch erfolgen . Der Knochenstoffwechsel ist sehr eng mit dem Auf- und Abbau von Knorpel verknüpft, wie die enchondrale Ossifikation oder die ähnlich ablaufende sekundäre Frakturheilung beweisen. Dementsprechend läßt sich auch die für die Konsolidierung von Knochenfragmenten mitentscheidende Kalzium-Ein- und Ausflußkinetik von Chondrozyten durch Pulsieren der elektromagnetischen Felder beeinflussen. Dabei macht sich insbesondere im Knorpel, der stark von der $O_2$-Diffusion abhängt, eine verstärkte Sauerstoffverfügbarkeit der Chondrozyten, die durch das Magnetfeld induziert wird, bemerkbar und führt zu einer erhöhten Syntheseleistung der Zelle. Durch eine formerhaltende und regenerationsfördernde Wirkung dieser elektrisch induzierten Knochenbildung gelingt es dem Organismus mit einem Minimum an Material und Energie die nötigen Strukturen aufzubauen. Eine Verletzung, Erkrankung oder bloße Verminderung der Elastizität eines Knochens führt zu einer Störung des Zellaufbaus, der Matrixproduktion und der Mineralisation. Durch pulsierende elektromagnetische Felder läßt sich die fehlende funktionelle Beanspruchung und der Verlust an Energie und Information kompensieren und die Knochenbildung und Frakturheilung deutlich beschleunigen.

Die Membrane der Membransysteme werden entweder direkt oder durch die im Kollagen gebildeten Potentiale beeinflußt oder erst über eine Änderung der Mikroumgebung der Zelle. Dieser Mechanismus basiert auf einer elektrochemischen Übertragung, die die Zellenaktivität durch Verschiebung der lonenatmosphäre im extra- und damit auch im intrazellulären Raum modifiziert. Die kapazitive Aufladung der Zellmembran durch die elektrische Komponente der pulsierenden elektromagnetischen Felder stellt dabei einen entscheidenden Faktor dar. Verursacht durch die Struktur-und Ladungsverschiebung in der Membran, insbesondere im Bereich der Poren, besteht die Möglichkeit einer Permeabilitätsänderung mit einer daraus resultierenden Beeinflussung passiver Ionentransport- und Diffusionsvorgänge. Durch die enge Kopplung von Oberflächenreaktion und Transmembrantransport scheinen vor allem aktive Transportsysteme, wie die Na-K-Pumpe, einen wichtigen Ansatzpunkt für die induzierte Energie darzustellen. Dabei kann eine gesteigerte Na-K-Adenosintriphosphatase-Aktivität eine verstärkte Natriumzufuhr durch die zuständige lonenpumpe bewirken. Dabei führt nur die Anregung mit einem optimalen, erfindungsgemäßen Amplitudenverlauf der Einzelimpulse über vermutlich eine Erhöhung der Oberflächenkonzentration der entsprechenden Ionen zur Anregung der aktiven Transportkomplexe.

Besonders gute Ergebnisse bei der Anregung des Stoffwechselaustausches werden erzielt, wenn der Verlauf jedes Einzelimpulses folgender Funktion entspricht:

$$y = \frac{x^3 \cdot e^{sin(x^3)}}{c}$$

Mit den in dieser Formel verwendeten Parametern läßt sich eine für die meisten Gewebearten generell sehr gute Stimulation erzielen.

Eine Optimierung der Wirkung der erfindungsgemäßen Vorrichtung auf den Organismus kann durch eine Rückkopplung verbessert werden. Zu diesem Zweck können Sensoren verwendet werden, die einen oder mehrere verschiedener Körperparameter messen, um die Anregung des Körpers durch die elektromagnetischen Pulse ' zu optimieren. Mit den Sensoren lassen sich beispielsweise Blutdruck, Temperatur, Puls oder Atemvolumen erfassen und zur Optimierung der Parameter der Vorrichtung zur Erzeugung elektromagnetischer Strahlung verwenden.

Weitere vorteilhafte Ausgestaltungen der Erfindung sind in den Unteransprüchen angegeben.

Anhand der Zeichnung wird die Erfindung näher erläutert. Es zeigen

Fig. 1 eine schematische Darstellung einer Ausführungsform der erfindungsgemäßen Vorrichtung zur Beeinflussung biologischer Abläufe;

Fig. 2 den zeitlichen Verlauf eines Einzelimpulses;

Fig. 3 den zeitlichen Ablauf mehrerer Einzelimpulse innerhalb von Impulsgruppen.

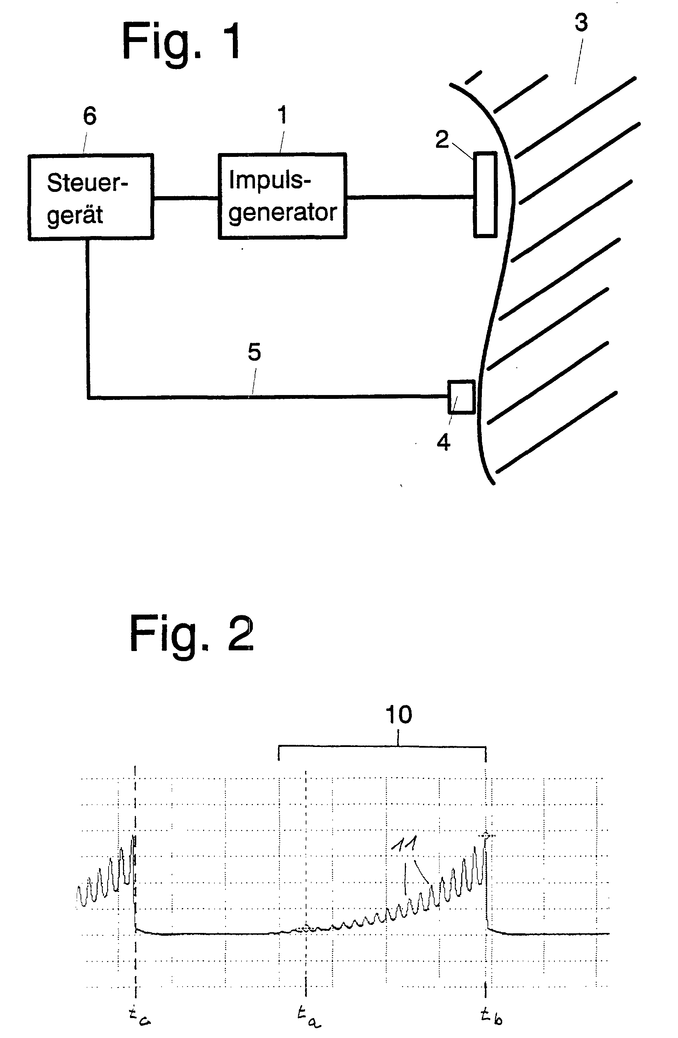

Wie in Fig. 1 dargestellt, besteht eine erfindungsgemäße Vorrichtung zumindest aus einem Impulsgenerator 1, der über eine Felderzeugungsvorrichtung bzw. Spule 2 ein pulsierendes elektromagnetisches Feld erzeugt, das in dem lebenden Gewebe 3, insbesondere im Körper eines zu behandelnden Patienten, zur Wirkung kommt. Zur Einstellung, insbesondere Optimierung, der Impulsparameter des pulsierenden elektromagnetischen Feldes im Generator 1 kann ein Sensor 4 bestimmte Körperparameter erfassen. Zu solchen Körperparametern zählen beispielsweise die Temperatur, der Blutdruck, die Pulsfrequenz oder der Sauerstoffgehalt des Blutes. Der erfaßte Parameter wird über eine Rückkopplungsleitung 5 einem Steuergerät 6 zugeführt, das den Parameter auswertet und den Impulsgenerator 1 entsprechend steuert. Für eine verbesserte Optimierung ist es möglich, mehrere Körperparameter zur Optimierung des pulsierenden Magnetfeldes gleichzeitig zu erfassen und auszuwerten.

Außerdem kann ein Sensor 4 zur Erfassung der durch den bestrahlten Körper übertragenen Frequenzen der Felderzeugungsvorrichtung 2 vorgesehen werden. Die erfaßten Frequenzen werden über die Leitung 5 dem Steuergerät 6 zugeführt. Aus den Unterschieden, insbesondere in der spektralen Zusammensetzung, zwischen den von der Felderzeugungsvorrichtung erzeugten Frequenzen und den vom Sensor 4 erfaßten Frequenzen berechnet das Steuergerät 6 die Übertragungsfunktion des bestrahlten Körpers. In Abhängigkeit von dieser Übertragungsfunktion legt das Steuergerät 6 die jeweils optimalen Werte für die Parameter a, b, c, d und k fest.

Bei solchen Felderzeugungsvorrichtungen 2 können die Feldstärken zusätzlich innerhalb der Geometrie der Felderzeugsvorrichtung 2 variiert werden.

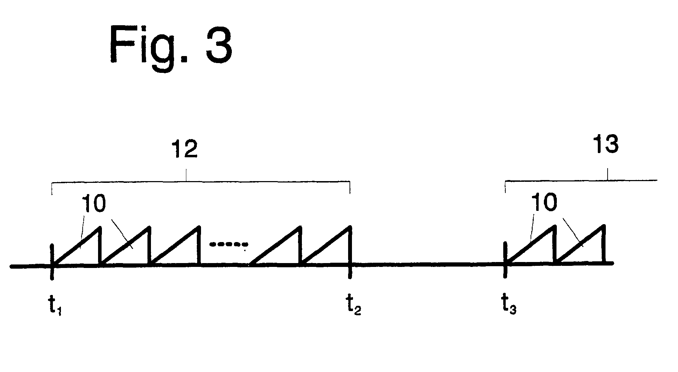

Mit dem Verfahren und der Vorrichtung der Erfindung wird ein pulsierendes elektromagnetisches Feld derart erzeugt, daß Einzelimpulse erzeugt werden, deren Form prinzipiell dem in Fig. 2 dargestellten Verlauf entsprechen. In Fig. 2 ist über der Zeit die Amplitude eines Einzelimpulses 10 und des Zeitraums zwischen zwei aufeinanderfolgenden Einzelimpulsen dargestellt. Die Einzelimpulse 10, aus denen sich das gepulste elektromagnetische Feld zusammensetzt, beginnen zu einem Zeitpunkt $t_a$ mit geringer Amplitude. Zum Ende des Einzelimpulses 10 zum Zeitpunkt $t_b$ hin nimmt die (mittlere) Amplitude beständig zu. Die Zunahme der Amplitude erfolgt vorzugsweise gemäß einer Potenzfunktion. Es sind aber auch andere Funktionen denkbar, die den (mittleren) Anstieg der Amplitude eines Einzelimpulses 10 über der Zeit beschreiben. Die optimale Form und Abfolge der Unterimpulse ist individuell sehr verschieden. Sie hängt von der Art des bestrahlten Gewebes, von dem erwünschten Heilungserfolg und von dem jeweiligen Individuum ab. Es hat sich gezeigt, daß die optimale Grundfrequenz dabei zwischen 1 und 1000 Hz schwanken kann. Zwischen den Einzelimpulsen 10 kann sich eine "Ruhezeit" bestimmter Länge befinden, die vermutlich aufgrund der Relaxationszeit der Austauschprozesse erforderlich ist. Das Tastverhältnis zwischen Ruhezeit (Zeitpunkte $t_c$ bis $t_a$ in Fig. 2) und aktiver Impulszeit ($t_a$ bis $t_b$) kann zwischen 3:1 bis 1:3 schwanken, vorzugsweise beträgt es in etwa 1:1. In den meisten Anwendungsfällen ist die Ruhepause jedoch entbehrlich.

Jedem Einzelimpuls 10 sind dabei zusätzliche Impulse (im folgenden auch als Unterimpulse bezeichnet) 11 überlagert. Zu Beginn jedes Einzelimpulses 10 beginnt die Amplitude der überlagerten Impulse 11 mit Null oder einem zuvor ausgewählten Offsetwert und etwa am Ende des Einzelimpulses zum Zeitpunkt $t_b$ erreicht sie die Maximalamplitude eines Einzelimpulses 10 (oder umgekehrt). Zwischen dem Zeitpunkt $t_a$ und dem Zeitpunkt $t_b$ nimmt die Amplitude der überlagerten Unterimpulse 11 kontinuierlich zu, ab oder ändert sich beliebig. Diese überlagerten Unterimpulse 11 führen zur Stimulation der physiologischen Austauschprozesse und tragen damit entscheidend zur Beschleunigung der angesprochenen Heilungsvorgänge bei. Wichtig ist dabei insbesondere, daß die Amplitude dieser Unterimpulse 11 im Verlauf eines jeden Einzelimpulses 10 variiert.

Eine entscheidende Bedeutung bei der Stimulation der Austauschprozesse im Körpergewebe hat vermutlich der hohe Anteil der an- bzw. absteigenden Flankenabschnitte, die durch die Vielzahl der überlagerten Unterimpulse 11 erzeugt wird.

Erfindungsgemäß nimmt die Steilheit des Anstiegs der überlagerten höherfrequenten Impulse 11 vom Beginn eines Einzelimpulses 10 bis zum Ende eines Einzelimpulses 10 zu. Dabei kann die Steilheit bei direkt aufeinanderfolgenden überlagerten Impulsen 11 in Abhängigkeit vom Faktor k bzw. k(x) auch kurzzeitig abnehmen.

Erfindungsgemäß weist jeder der Einzelimpulse beispielsweise einen Verlauf auf, der der folgenden Funktion entspricht:

$$y = \frac{x^a \cdot k \cdot e^{sin(x^b)}}{c} + d$$

Diese Formel gibt für jeden Einzelimpuls 10 den Verlauf der Amplitude y über der Zeit x an. Die Zeit x fängt für jeden Einzelimpuls 10 zu Beginn dieses Impulses wieder mit demselben Anfangswert, vorzugsweise 0, an. Diese Formel gibt nur den Verlauf der "aktiven" Impulszeit an. Die jeweils vorzusehenden Ruhepausen zwischen den Einzelimpulsen werden von dieser Formel nicht angegeben. In der Zeit zwischen den Einzelimpulsen nimmt das Signal bzw. das pulsierende elektromagnetische Feld den Wert 0 oder einen voreinstellbaren festen Wert an. Das bedeutet, der Bereich, den der Parameter x durchläuft, hängt von der Dauer der "aktiven" Impulsdauer ab. Dieser wird von der Frequenz der Einzelimpulse, die zwischen 1 Hz und 1000 Hz liegen kann, und dem Tastverhältnis zwischen "aktiver" und "passiver" Impulsdauer bestimmt. Die Zeitvariable x variiert vorzugsweise zwischen -4,5 und +4,5, wobei für die meisten Anwendungen der Bereich von 0 bis in etwa +4 (also 0 bis 3 - 4) ausreichend ist.

Der Exponent a gibt an, mit welcher Steilheit die Amplitude des Grundimpulses während der "aktiven" Impulsdauer zunimmt. Durch diesen Parameter wird sozusagen eine Art "Hüllkurve" für den tatsächlichen Verlauf jedes Einzelimpulses festgelegt. Bevorzugte Werte für a liegen im Bereich von a = 1 bis a = 5, vorzugsweise wird der Wert 3 verwendet. Der Parameter a kann auch entsprechende negative Werte annehmen.

Der Exponent b beschreibt die Anzahl und Steilheit der dem durch den Parameter a festgelegten Grundimpulsverlauf überlagerten Unterimpulse 11. Je größer der Parameter b gewählt wird, desto mehr Unterimpulse 11 sind jedem Grundimpuls überlagert. Der Parameter b nimmt vorzugsweise Werte zwischen 2 und 5 an, im allgemeinen wird der Wert 3 verwendet.

Der Parameter c stellt eine Art Skalierungsfaktor dar. Mit ihm läßt sich ein maximaler Signal- bzw. Feldstärkewert jedes Einzelimpulses einstellen. Je größer der Parameter c gewählt wird, desto kleiner wird der erreichte maximale Amplitudenwert. Der Parameter c ist so gewählt, daß die in den einzelnen Ländern unterschiedlichen zulässigen elektromagnetischen Feldstärken eingehalten werden können. Die WHO schlägt Werte kleiner als 100 µT bei Daueranwendung im niederfrequenten Bereich vor. Der jeweils zu verwendende genaue Wert für c ist deshalb von der Charakteristik der verwendeten Spule bzw. Felderzeugungsvorrichtung 2 abhängig. Für spezielle Anwendungen kann der Parameter in Abhängigkeit von der Zeit, beispielsweise durch eine programmabhängige Steuerung, variieren. Bei ansonsten gleicher Impulsfrequenz kann das Signal z.B. zunächst eine Minute mit einer niedrigen, anschließend 2 Minuten mit einer höheren Feldstärke usw. betrieben werden.

Mit dem Parameter d läßt sich eine Art "Vorspannung" der Einzelimpulse 10 bestimmen, d.h. es läßt sich ein Grundsignalwert (Offsetwert) einstellen, auf dem die Einzelimpulse 10 jeweils "aufsetzen". Dieser Grundwert muß keinem fest gewählten Amplitudenwert entsprechen, sondern kann auch im Verlauf der Zeit variieren (Nulliniensymmetrie oder Nullinienasymmetrie). Dabei können durch geeignete Wahl elektrophoretische Vorgänge beeinflußt werden. Vorzugsweise wird ein Wert zwischen -1 und +2 verwendet, im allgemeinen 0. Der Parameter ist dabei insbesondere so zu wählen, daß der zulässige Feldstärkebereich nicht verlassen wird.

In einer bevorzugten Ausführungsform der Erfindung wird für den Parameter a der Wert 3 gewählt, für den Parameter b der Wert 3, für den Parameter k der Wert 1 und für den Parameter d der Wert 0 (der Parameter c ist in Abhängigkeit von den obigen Randbedingungen zu wählen und wird deshalb im folgenden nicht weiter mit angegeben). Wenn die Einzelimpulse 10 einen solchen Verlauf aufweisen, läßt sich eine besonders vorteilhafte Stimulierung der biologischen Abläufe erreichen. Der Amplitudenverlauf jedes Einzelimpulses 10 entspricht dann folgender Formel:

$$y = x^3 \cdot e^{sin(x^3)}$$

Wenn mit Hilfe von Sensoren bestimmte Parameter des lebenden Gewebes, insbesondere des menschlichen Körpers, erfaßt werden, läßt sich der Verlauf jedes Einzelimpulses 10 so an die tatsächlichen Verhältnisse anpassen, daß eine optimale Stimulation erreicht wird. Dazu werden in Abhängigkeit von den erfaßten Gewebeparametern (oder dem erfaßten Gewebeparameter) die Parameter des Impulsverlaufes, d. h. a, b, c, d und k, entsprechend verändert. Auf diese Weise ist eine adaptive Anpassung der Stimulation an die Sensibilität des zu stimulierenden Gewebes möglich. Der Umfang, in dem eine Variation in Abhängigkeit von Gewebeparametern möglich ist, hängt von der Gewebeart, der erwünschten Anregung und insbesondere von der physikalischen Qualität der erfaßten Gewebeparameter ab.

Wird ein Einzelimpulsverlauf gemäß der oben angegebenen besonders vorteilhaften Ausführungsform der Erfindung gewählt, so lassen sich über eine solche Rückkopplungsschleife die Parameter in geringem Umfang verändern, um eine Anpassung des Impulsverlaufs an eine beispielsweise durch die Anregung selbst verursachte Sensibilitätsänderung des beaufschlagten Gewebes auszugleichen.

Fig. 3 zeigt die Einzelimpulse 10 aus Fig. 2 in einem größeren zeitlichen Maßstab. Die Einzelimpulse 10 sind zu Impulsgruppen 12, 13 zusammengeschlossen, in denen eine Vielzahl von Einzelimpulsen aufeinander folgt. Zwischen dem Zeitpunkt $t_1$, der den Beginn einer solchen Impulsgruppe markiert, und dem Zeitpunkt $t_2$, der das Ende einer Impulsgruppe markiert, entspricht der zeitliche Verlauf eines jeden Impulses dem in Fig. 2 dargestellten Verlauf. Der Einfachheit halber ist der Amplitudenverlauf jedes Einzelimpulses in Fig. 3 mit einem Dreieck angedeutet. Die Dauer jeder Impulsgruppe liegt in Abhängigkeit von den äußeren Umständen zwischen 0,25 sec und 1,2 sec. Vorteilhafterweise wird die Dauer der Impulsgruppen während der Dauer der Beaufschlagung des Gewebes mit dem pulsierenden Magnetfeld in Abhängigkeit von der Zeit verändert. Dabei hat sich als besonders vorteilhaft herausgestellt, die Länge der Impulsgruppen mit zunehmender Beaufschlagungszeit zunehmen zu lassen. Zwischen solchen Impulsgruppen befindet sich eine Impulspause ($t_2$ bis $t_3$), die zwischen dem 0,05-fachen und dem dreifachen der Zeitdauer einer Impulsgruppe 12, 13 schwanken kann. Solche Impulspausen führen erfahrungsgemäß zu einer besseren Anregung des lebenden Körpergewebes.

**Patentansprüche**

1. Vorrichtung zur Beeinflussung biologischer Abläufe in einem lebenden Gewebe, insbesondere einem menschlichen Körper, durch Beaufschlagung zumindest eines Teils des Gewebes mit einem pulsierenden elektromagnetischen Feld, umfassend

   eine Felderzeugungsvorrichtung (2) zur Erzeugung des pulsierenden elektromagnetischen Feldes und

   einen Impulsgenerator (1) zur Ansteuerung der Felderzeugungsvorrichtung (2), gekennzeichnet daduch, dass der Impulsgenerator (1) die Felderzeugungsvorrichtung (2) so ansteuert,

   dass das pulsierende elektromagnetische Feld aus einer Vielzahl von Einzelimpulsen (10) besteht, deren Frequenz zwischen 1 Hz und 1000 Hz liegt,

   dass die Amplitude jedes Einzelimpulses (10) der folgenden Funktion entspricht:

$$y = \frac{x^a \cdot k \cdot e^{sin(x^b)}}{c} + d$$

wobei die Parameter jeweils angeben:

y   die Amplitude des erzeugten Signalverlaufs,

x   den Zeitverlauf, wobei die Zeit x für jeden Einzelimpuls (10) wieder von neuem mit demselben Anfangswert beginnt,

a   einen Parameter zur Einstellung des zeitlichen Amplitudenverlaufs jedes Einzelimpulses (10),

b   die Anzahl der überlagerten Impulse,

c   einen Faktor zur Einstellung der Amplitude,

d   einen Offsetwert und

k   einen Faktor zur Einstellung einer Amplitude der überlagerten Impulse.

2.  Vorrichtung nach Anspruch 1, **dadurch gekennzeichnet,** dass ein modulierter Einzelimpuls (10) des pulsierenden elektromagnetischen Feldes der folgenden Funktion entspricht:

$$y = \frac{x^3 \cdot e^{sin(x^3)}}{c}$$

3.  Vorrichtung nach Anspruch 1 oder 2, **dadurch gekennzeichnet,** dass die Einzelimpulse (10) in Impulsgruppen (12,13) auftreten, wobei die Dauer jeder Impulsgruppe (12,13) zwischen 0,25 Sekunden und 1,2 Sekunden liegt.

4.  Vorrichtung nach Anspruch 3, **dadurch gekennzeichnet,** dass die Dauer der Impulsgruppen (12,13) während der Dauer der Beaufschlagung des Gewebes mit dem pulsierenden elektromagnetischen Feld in Abhängigkeit von der Zeit variiert.

5.  Vorrichtung nach Anspruch 3 oder 4, **dadurch gekennzeichnet,** dass das Taktverhältnis zwischen den Einzelimpulsen (10) und den dazwischen liegenden Ruhepausen innerhalb der Impulsgruppen (12,13) 3:1 bis 1:3 beträgt.

6.  Vorrichtung nach einem der Ansprüche 1 bis 5, **dadurch gekennzeichnet,** dass die Vorrichtung außerdem umfasst:

mindestens einen Sensor (4) zur Erfassung jeweils eines Gewebeparameters und

ein Steuergerät (6), dem der von dem mindestens einen Sensor (4) erfasste Gewebeparameter zugeführt wird, zur Optimierung des Verlaufs des pulsierenden Magnetfeldes durch Auswertung der erfassten Gewebeparameter und Ansteuerung des Impulsgenerators (1).

**Claims**

1.  A device for influencing biological processes in a living tissue, particularly in a human body, by subjecting at least a part of the tissue to the action of a pulsating electromagnetic field, comprising

a field-generating device (2) for generating the pulsating electromagnetic field and

a pulse generator (1) for driving the field-generating device (2), **characterised in that** the pulse generator (1) drives the field-generating device (2) in such a way

that the pulsating electromagnetic field consists of a plurality of individual pulses (10), the frequency of which lies between 1 and 1,000 Hz,

that the amplitude of each individual pulse (10) corresponds to the following function:

$$y = \frac{x^a \cdot k \cdot e^{sin(x^b)}}{c} + d$$

whereby the parameters specify the following:

y   the amplitude of the signal curve generated,

x   the course of time, whereby the time *x* recommences anew with the same initial value for each individual pulse (10),

a   a parameter for setting the temporal amplitude response of each individual pulse (10),

b   the number of superimposed pulses,

c   a factor for setting the amplitude,

d   an offset value and

k   a factor for setting an amplitude of the superimposed pulses.

2.  Device according to Claim 1, **characterised in that** a modulated individual pulse (10) of the pulsating electromagnetic field corresponds to the following function:

$$y = \frac{x^3 \cdot e^{sin(x^3)}}{c}$$

**3.** Device according to Claim 1 or 2, **characterised in that** the individual pulses (10) occur in pulse groups (12, 13), the duration of each pulse group (12, 13) being between 0.25 seconds and 1.2 seconds.

**4.** Device according to Claim 3, **characterised in that** the duration of the pulse groups (12, 13) during the period in which the tissue is subjected to the action of the pulsating electromagnetic field varies as a function of time.

**5.** Device according to Claim 3 or 4, **characterised in that** the mark-to-space ratio of the individual pulses (10) to the rest periods between them within the pulse groups (12, 13) amounts to 3:1 to 1:3.

**6.** Device according to one of Claims 1 to 5, **characterised in that** the device comprises, in addition:

at least one sensor (4) for recording, in each instance, a tissue parameter and

a control unit (6), to which the tissue parameter recorded by the at least one sensor (4) is supplied, for the purpose of optimizing the curve of the pulsating magnetic field by evaluating the recorded tissue parameters and driving the pulse generator (1).

**Revendications**

**1.** Dispositif pour influencer des processus biologiques dans un tissu vivant, en particulier dans le corps humain, par application d'un champ électromagnétique pulsé à au moins une partie du tissu, comprenant

un dispositif de génération de champ (2) destiné à produire le champ électromagnétique pulsé, et
un générateur d'impulsions (1) pour commander le dispositif de génération de champ (2), **caractérisé** en
ce que le générateur d'impulsions (1) commande le dispositif de génération de champ (2) de telle sorte

que le champ électromagnétique pulsé soit formé d'une pluralité d'impulsions individuelles (10) dont la fréquence est comprise entre 1 et 1000 Hz,
que l'amplitude de chaque impulsion individuelle (10) suive la fonction suivante :

$$y = \frac{x^a \cdot k \cdot e^{sin(x^b)}}{c} + d \,,$$

y étant l'amplitude du signal produit,
x, l'évolution du temps, le temps x reprenant la même valeur initiale pour chaque impulsion individuelle (10),

a, un paramètre destiné à régler l'évolution de l'amplitude en fonction du temps de chaque impulsion individuelle (10),
b, le nombre d'impulsions superposées,
c, un facteur de réglage de l'amplitude,
d, une valeur de décalage, et
k, un facteur de réglage d'une amplitude des impulsions superposées.

**2.** Dispositif selon la revendication 1, **caractérisé** en ce qu'une impulsion individuelle (10) modulée du champ électromagnétique pulsé suit la fonction suivante :

$$y = \frac{x^3 \cdot e^{sin(x^3)}}{c}$$

**3.** Dispositif selon la revendication 1 ou 2, **caractérisé** en ce que les impulsions individuelles (10) apparaissent sous forme de groupes d'impulsions (12, 13), la durée de chaque groupe d'impulsions (12, 13) étant comprise entre 0,25 et 1,2 seconde.

**4.** Dispositif selon la revendication 3, **caractérisé** en ce que la durée des groupes d'impulsions (12, 13) varie en fonction du temps pendant la durée de l'application du champ électromagnétique pulsé au tissu.

**5.** Dispositif selon la revendication 3 ou 4, **caractérisé** en ce que le rapport de rythme entre les impulsions individuelles (10) et les pauses intermédiaires à l'intérieur des groupes d'impulsions (12, 13) est de 3:1 à 1:3.

**6.** Dispositif selon une des revendications 1 à 5, **caractérisé** en ce que le dispositif comprend en outre :

au moins un capteur (4) pour détecter un paramètre du tissu, et
un appareil de commande (6) auquel est transmis le paramètre du tissu détecté par le capteur (4), au nombre d'au moins un, pour optimiser la configuration
du champ magnétique pulsé par exploitation du paramètre de tissu détecté et commande du générateur d'impulsions (1).

# Fig. 1

# Fig. 2

# Fig. 3